# FASCICULE DE BREVET EUROPEEN

(11) **EP 2 413 880 B1**
(45) Date de publication et mention de la délivrance du brevet: **06.05.2015**
(21) Numéro de dépôt: 09784370.0
(22) Date de dépôt: 31.03.2009
(51) Int. Cl.: A61Q 5/12, A61K 8/73, A61K 8/898, A61K 8/96

(54) **COMPOSITION CAPILLAIRE DE TRAITEMENT DES CHEVEUX**
KAPILLARZUSAMMENSETZUNG ZUR HAARBEHANDLUNG
CAPILLARY COMPOSITION FOR TREATING HAIR

(43) Date de publication de la demande: 08.02.2012
(73) Titulaire: L'Oréal, 75008 Paris (FR)
(72) Inventeur: KHENNICHE, Samira, F-92110 Clichy (FR); ROLLAT-CORVOL, Isabelle, F-75017 Paris (FR); BOUREL, Sophie, F-92150 Suresnes (FR)
(74) Mandataire: Le Coupanec, Pascale A.M.P.
(86) Numéro de dépôt international: PCT/FR2009/050551
(87) Numéro de publication internationale: WO 2010/112683

(56) Documents cités:
- EP-A1- 0 530 974
- DE-A1-102005 060 435
- US-A1- 2002 012 697
- US-A1- 2006 234 886
- US-A1- 2007 140 985

## Description

La présente invention est relative au traitement des cheveux humains.

Les cheveux peuvent être altérés par des traitements chimiques tels que la coloration, la permanente, ou par des sollicitations mécaniques telles que le démêlage, le brushing. Les propriétés mécaniques, morphologiques et physicochimiques de la surface des cheveux, et notamment de la cuticule, couche externe du cheveu avec une structure en écailles, s'en trouvent modifiées. En particulier, au cours de ces traitements ou sollicitations, les écailles de la cuticule se soulèvent et voient leurs bordures, normalement régulières, devenir dentelées. Ces détériorations peuvent avoir plusieurs conséquences. D'une part, les cheveux sont moins lisses et moins facilement démêlables. D'autre part, les actifs d'un produit de traitement des cheveux, par exemple d'un conditionneur, risquent d'être déposés de façon hétérogène sur le cheveu. Or, il peut s'avérer souhaitable de déposer les actifs de soin de manière homogène sur tout le cheveu.

Il est connu de soigner des cheveux abîmés en appliquant sur ceux-ci un produit de soin comprenant par exemple des polymères spécifiques tels que des silicones ou des polymères à charges cationiques. Cependant, l'amélioration des cheveux ainsi traités n'est que provisoire car une fois débarrassés du produit de soin, par exemple après un ou plusieurs lavages, les cheveux retrouvent leur état d'origine.

Il existe un besoin de compositions, de procédés et de kits permettant de traiter durablement les cheveux, en particulier ceux abîmés en surface.

L'invention vise entre autres à répondre à ce besoin et elle y parvient grâce à un procédé de traitement des cheveux mettant en oeuvre une composition capillaire comprenant au moins des particules de pierre ponce, une ou plusieurs silicones à groupements ammonium quaternaire et un ou plusieurs polymères non siliconés.

Un premier objet de l'invention est donc une composition capillaire comprenant au moins des particules de pierre ponce, une ou plusieurs silicones à groupements ammonium quaternaire et un ou plusieurs polymères non siliconés.

Un second objet de l'invention est un procédé de traitement des cheveux comportant au moins l'étape consistant à mettre en contact les cheveux avec une composition capillaire comme indiquée ci-dessus.

Comme il ressort des exemples ci-après, le traitement des fibres kératiniques, et notamment des cheveux, avec une composition conforme à l'invention permet d'apporter du soin de manière homogène à la fibre kératinique sans l'altérer en uniformisant le dépôt des agents traitants. On obtient ainsi un excellent lissage des fibres kératiniques et un volume de la chevelure maitrisé. Ces effets sont d'autant plus remarquables que les cheveux sont sensibilisés et/ou épais.

Ces effets sont durables et résistent notamment au(x) shampooing(s).

Le procédé peut comporter également l'étape consistant à peigner et/ou rincer les cheveux à l'issue dudit traitement.

Le procédé peut être également avantageusement mis en oeuvre pour lisser les cheveux.

L'invention peut également permettre de préparer les cheveux à un post-traitement capillaire tel que l'application d'un conditionneur, d'une coloration, d'une permanente, d'un produit de défrisage, d'un produit de décoloration ou autre.

Le procédé peut ainsi comporter l'étape consistant à soumettre les cheveux à un post-traitement, après traitement à l'aide de la composition capillaire de l'invention, le post-traitement étant choisi parmi l'application d'un conditionneur, d'une permanente, d'un défrisant, d'un produit de coloration ou de décoloration des cheveux, cette liste n'étant pas limitative.

Le traitement des cheveux qui est opéré en mettant en oeuvre l'invention peut être plus ou moins important, en fonction de l'état initial des cheveux et/ou du résultat recherché. Ce traitement peut notamment avoir pour effet d'inclure une élimination des hétérogénéités figurant en surface des cheveux, notamment *via* une action que l'on peut qualifier d'abrasive et, de ce fait, homogénéiser la surface externe des cheveux.

Ce type d'abrasion peut être relativement doux et/ou de courte durée, de manière à éviter une cassure des cheveux, lors du traitement ou de sollicitations mécaniques ultérieures telles que le coiffage, par exemple.

Grâce à l'invention, les cheveux peuvent être visiblement plus lisses et l'effet du traitement est durable.

Sans être lié par une quelconque théorie on peut penser que grâce à l'invention, les cheveux peuvent être débarrassés de dépôts éventuellement présents à leur surface avant l'abrasion et les bordures des écailles de la cuticule peuvent être rendues plus régulières, ce qui permet une efficacité accrue de la ou des silicones à groupements ammonium quaternaire.

De plus, après le traitement, des produits destinés à renforcer certaines propriétés des cheveux ou à modifier leur aspect peuvent pénétrer plus facilement et profondément dans les cheveux ainsi traités.

### Composition capillaire

### Particules de pierre ponce

La pierre ponce (nom INCl : pumice) est d'origine volcanique. Elle se forme à des températures d'environ 500 à 600 °C à partir de la lave projetée en l'air qui se refroidit dans sa chute et dont le dégazage entraîne la formation de bulles dont résultent une densité faible et une porosité importante.

La pierre ponce est formée de fragments de rhyolite, de dacite ou d'andésite. Elle est considérée comme un verre car elle n'a pas de structure cristalline.

Les particules de pierre ponce sont des particules solides abrasives. En particulier, elles peuvent avoir une dureté supérieure ou égale à celle des cheveux, allant de 3 à 10 Mohs, voire supérieure ou égale à 4, par exemple supérieure ou égale à 5, et en particulier allant de 5 à 5,5 sur l'échelle de Mohs.

Les particules de pierre ponce peuvent avoir un diamètre moyen en volume inférieur ou égal à 500 µm, de préférence compris entre 50 et 500 µm, mieux inférieur ou égal à 300 µm, par exemple compris entre 100 et 300 µm.

Suivant la gamme de particules mises en oeuvre, le diamètre moyen en volume peut être déterminé par utilisation de tamis ou par granulométrie laser.

Il peut notamment s'agir d'une poudre de pierre ponce commercialisée sous le nom de PONCE 0 ½ D par la société EYRAUD, de diamètre moyen D [4,3] (diamètre moyen pondéré en volume) d'environ 140 µm mesuré par diffraction laser.

Il peut également s'agir d'une poudre de pierre ponce décontaminée commercialisée sous la référence 0-D PONCE par la Société EYRAUD, de diamètre moyen en volume inférieur à 125 µm, ou encore d'une poudre de pierre ponce commercialisée sous la référence 2B D par la Société EYRAUD, de diamètre moyen en volume allant de 100 à 500 µm.

La composition capillaire conforme à l'invention peut comporter des particules de pierre ponce en une teneur allant de 0,1 % à 35 % en poids, notamment de 5 % à 30 % en poids, en particulier de 10 % à 25 % en poids, par exemple de 15 % à 20 % en poids, mieux de 18 % à 20 % en poids par rapport au poids total de la composition.

### Silicones à groupements ammonium Quaternaire

Conformément à l'invention, on entend par « silicone à groupements ammonium quaternaire » toute silicone comportant un ou plusieurs groupements ammonium quaternaire. Ces groupements ammonium quaternaire peuvent être liés en position alpha ou oméga ou sous forme de groupements latéraux. Ils peuvent être liés directement au squelette polysiloxane ou peuvent être portés par des chaînes hydrocarbonées.

Selon l'invention, on entend par silicone, en conformité avec l'acceptation générale, tout polymère ayant une structure basée sur l'alternance d'atomes de silicium et d'oxygène, reliés entre eux par des liaisons dites liaisons siloxane (-Si-O-Si-), et caractérisée en outre par l'existence de liaisons silicium-carbone. Ces silicones, ou polysiloxanes, sont généralement obtenues par polycondensation de silanes convenablement fonctionnalisés. Les radicaux hydrocarbonés les plus courants portés par les atomes de silicium sont les radicaux alkyle inférieurs, en particulier méthyle, les radicaux fluoroalkyles, les radicaux aryle et en particulier phényle.

Les silicones à groupements ammonium quaternaire de la présente invention sont par exemple choisies parmi les composés correspondants aux formules générales suivantes : 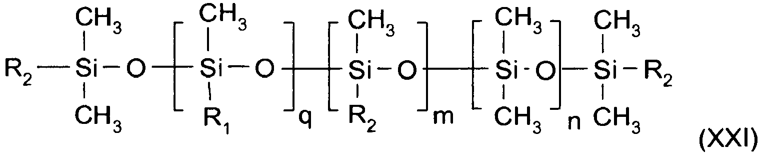 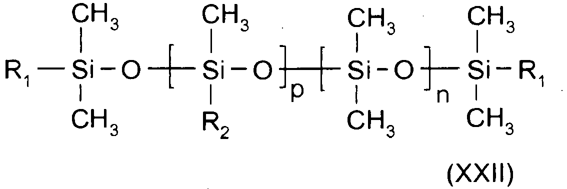 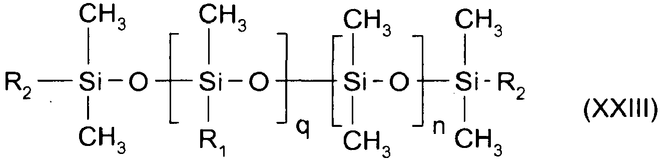 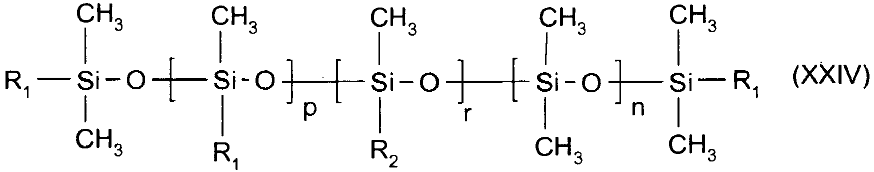 dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-(PO₃H)_{d}-R₅ ou -C_{c}H_{2c}-O-(C₄H₈O)ₐ-(PO₃H)_{d} -R₅ ;
   R₅, identique ou différent, est choisi parmi les groupes de formule suivante : 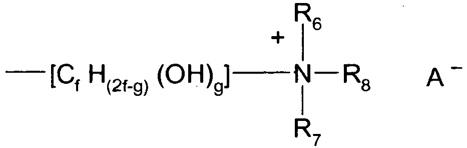
- les radicaux R₈ représentent indépendamment un radical alkyle en C₁₋₂₂ ou alcényle en C_{1-22'} linéaire ou ramifié, et portant éventuellement un ou plusieurs groupements OH, ou représentent un groupement CₕH₂ₕZCOR₉; et
- R₆, R₇ et R₉, identiques ou différents, représentent des radicaux alkyle en C₁-₂₂ ou alcényle en C₁-_{22'}, linéaires ou ramifiés, portant éventuellement un ou plusieurs groupements OH, ou R₇ peut former avec une partie de R₈ un hétérocycle (cycle avec au moins un hétéroatome tel que par exemple N, O, P), l'hétérocycle est notamment une imidazoline.

De préférence R₆ et R₇ désignent un radical alkyle en C₁-C₆ et plus particulièrement méthyle, R₉ désigne de préférence un radical choisi parmi les alkyle en C₈-C₁₈ et les alcényle en C₈-C₁₈ et notamment un radical cocoyle.
- m varie de 0 à 20;
- n varie de 0 à 500 ;
- p varie de 1 à 50 ;
- q varie de 0 à 20 ;
- r varie de 1 à 20 ;
- a varie de 0 à 50 ;
- b varie de 0 à 50 ;
- c varie de 0 à 4 ;
- d désigne 0 ou 1 ;
- f varie de 0 à 4 ;
- g varie de 0 à 2, de préférence est égal à 1 ; et
- h varie de 1 à 4, de préférence est égal à 3 ;
   Z représente un atome d'oxygène ou NH,
   A⁻ représente un anion minéral ou organique monovalent tel qu'un halogénure (par exemple chlorure, bromure), un sulfate, ou un carboxylate (par ex. acétate, lactate, citrate).

De préférence on utilise les silicones à groupements ammonium quaternaire de formule (XXII) ou (XXIII).

De préférence, on utilise les silicones à groupements ammonium quaternaire répondant à la formule générale (XXIII) telle que définie ci-dessus, et plus particulièrement celles répondant à la formule générale (XXIII) dans laquelle au moins l'une des, et de préférence toutes les conditions suivantes sont satisfaites :
- c est égal à 0;
- d désigne 0 ;
- a est égal à zéro ;
- b est égal à 1
- n varie de 0 à 100 ;
- q est égal à 0 ;
- f = 3 ;
- g = 1 ;
- R₆ et R₇ désignent le groupe méthyle ; et
- R₈ désigne un radical alkyle C₁₀-C₂₂.

Parmi les silicones de l'invention, on peut citer par exemple celles commercialisées par la société GOLDSCHMIDT sous les dénominations ABIL QUAT 3272, ABIL B 9905, ABIL QUANT 3474 et ABIL K 3270, par la société LIPO FRANCE sous les dénominations SILQUAT Q-100, SILQUAT Q-200 WS, SILQUAT AX, SILQUAT AC, SILQUAT AD et SILQUAT AM tous fabriqués par la société SILTECH, par la société OSI sous la dénomination MAGNASOFT EXHAUST et SILSOFT C-880 et par la société UCIB sous les dénominations PECOSIL 14-PQ et PECOSIL 36-PQ (fabricant PHOENIX CHEMICAL). Ces silicones sont notamment décrites dans les brevets EP 530 974, DE 3 719 086, DE 3 705 121, EP 617 607 et EP 714 654.

Selon un mode de réalisation, la silicone à groupements ammonium quaternaire est de formule (XXIII). Encore plus préférentiellement la silicone à groupements ammonium quaternaire est le composé référencé au CTFA (nom INCI) sous l'appellation Quaternium-80.

Les silicones à groupements ammonium quaternaire utilisées conformément à l'invention peuvent se présenter sous forme de solutions aqueuses ou éventuellement sous forme de dispersions ou d'émulsions dans l'eau.

La composition capillaire peut comporter des silicones à groupements ammonium quaternaire en une teneur allant de 0,1 à 20 % en poids, de préférence de 0,2 à 10 % en poids, mieux de 0,3 à 5 % en poids par rapport au poids total de la composition.

Le rapport pondéral de la quantité de particules de pierre ponce à la quantité de silicones à groupements ammonium quaternaire varie de préférence de 1 à 250, encore plus préférentiellement de 5 à 200, mieux de 10 à 100.

### Polymère non siliconé

La composition capillaire de l'invention comprend en outre un ou plusieurs polymères non siliconés.

Par polymère non siliconé, on entend au sens de la présente invention tout polymère ne comportant pas d'atome de silicium dans sa structure et qui comporte dans ladite structure la répétition d'au moins un motif autre qu'un motif oxyde d'alkylène ou glycérol. 1.

Le ou les polymères non siliconés peuvent être choisis parmi les polymères épaississants, fixants ou conditionneurs, non ioniques, anioniques, cationiques ou amphotères.

Par polymère épaississant, on entend au sens de la présente invention tout polymère qui par sa présence permet d'augmenter la viscosité de la composition d'au moins 20 centipoises à 25 °C et à un taux de cisaillement de 1s⁻¹. Encore plus préférentiellement on entend par polymère épaississant un polymère qui, introduit à 1 % en poids dans une solution aqueuse ou hydroalcoolique à 30 % d'éthanol, et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25 °C et à un taux de cisaillement de 1s⁻¹. Cette viscosité peut être mesurée à l'aide d'un viscosimètre cône/plan (Rhéomètre Haake R600 ou analogue).

Par polymère fixant, on entend au sens de la présente invention tout polymère capable de conférer une forme à une chevelure ou de permettre le maintien d'une forme acquise.

Par polymère conditionneur on entend au sens de la présente invention tout polymère qui par sa présence permet d'améliorer l'état cosmétique des fibres kératiniques en particulier au niveau du toucher ou des propriétés de démêlage.

Les polymères de l'invention peuvent être non ioniques, anioniques, cationiques ou amphotères.

Plus préférentiellement la composition de l'invention comprend un ou plusieurs polymères épaississants. Encore plus préférentiellement les polymères épaississants de l'invention sont des polymères épaississants de phase aqueuse non ioniques, anioniques, cationiques ou amphotères.

Les polymères épaississants peuvent être des polymères associatifs ou non.

A titre de polymères épaississants selon l'invention on peut citer les polymères épaississants à motifs sucres.

Par motif sucre on entend au sens de la présente invention un motif issu d'un carbohydrate de formule Cₙ(H₂O)ₙ₋₁ ou (CH₂O)ₙ pouvant être éventuellement modifié par substitution et/ou par oxydation et/ou par déshydratation.

Les motifs sucre pouvant entrer dans la composition des polymères épaississants de l'invention sont de préférence issus des sucres suivants : glucose, galactose, arabinose, rhamnose, mannose, xylose, fucose, anhydrogalactose, acide galacturonique, acide glucuronique, acide mannuronique, galactose sulfate anhydrogalactose sulfate.

On peut notamment citer à titre de polymères épaississants de l'invention :
- les gommes natives telles que :
   a) les exsudats d'arbres ou d'arbustes dont :
      - la gomme arabique (polymère ramifié de galactose, d'arabinose, de rhamnose et d'acide glucuronique) ;
      - la gomme ghatti (polymère issu d'arabinose, de galactose, de mannose, de xylose et d'acide glucuronique) ;
      - la gomme karaya (polymère issu d'acide galacturonique, de galactose, de rhamnose et d'acide glucuronique) ; et
      - la gomme tragacanthe (ou adragante) (polymère d'acide galacturonique, de galactose, de fucose, de xylose et d'arabinose).
   b) les gommes issues d'algues dont :
      - l'agar (polymère issu de galactose et d'anhydrogalactose) ;
      - les alginates (polymères d'acide mannuronique et d'acide glucuronique) ; et
      - les carraghénanes et les furcelleranes (polymères de galactose sulfate et d'anhydrogalactose sulfate).
   c) les gommes issus de semences ou tubercules dont :
      - la gomme de guar (polymère de mannose et de galactose) ;
      - la gomme de caroube (polymère de mannose et de galactose) ;
      - la gomme de fenugrec (polymère de mannose et de galactose) ;
      - la gomme de tamarin (polymère de galactose, de xylose et de glucose) ;
      - la gomme de konjac (polymère de glucose et mannose) ;
   d) les gommes microbiennes dont :
      - la gomme de xanthane (polymère de glucose, de mannose acétate, de mannose/acide pyruvique et d'acide glucuronique) ;
      - la gomme de gellane (polymère de glucose partiellement acylé, de rhamnose et d'acide glucuronique) ; et
      - la gomme de scléroglucane (polymère du glucose) ;
   e) les extraits de plantes dont :
      - la cellulose (polymère du glucose) ; et
      - l'amidon (polymère du glucose).

Ces polymères peuvent être modifiés par voie physique ou chimique. A titre de traitement physique on peut citer notamment la température.

A titre de traitements chimiques on peut citer les réactions d'estérification, d'étherification, d'amidification, d'oxydation. Ces traitements permettent de conduire à des polymères qui peuvent être notamment non ioniques, anioniques ou amphotères.

De préférence ces traitements chimiques ou physiques sont appliqués sur les gommes de guar, les gommes de caroube, les amidons et les celluloses.

Les gommes de guar non-ioniques utilisables selon l'invention peuvent être modifiées par des groupements hydroxylakyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société RHODIA CHIMIE.

Les molécules d'amidons utilisées dans la présente invention peuvent avoir comme origine botanique les céréales ou encore les tubercules. Ainsi, les amidons sont par exemple choisis parmi les amidons de maïs, de riz, de manioc, d'orge, de pomme de terre, de blé, de sorgho, de pois.

Les amidons peuvent être modifiés par voie chimique ou physique : notamment par une ou plusieurs des réactions suivantes : prégélatinisation, oxydation, réticulation, estérification, éthérification, amidification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C₁-C₆ (acétyl), hydroxyalkylés en C₁-C₆ (hydroxyéthyl, hydroxypropyl), carboxyméthyl, octénylsuccinique.

On peut notamment obtenir par réticulation avec des composés phosphorés des phosphates de monoamidon (du type Am-O-PO-(OX)₂), des phosphates de diamidon (du type Am-O-PO-(OX)-O-Am) ou même de triamidon (du type Am-O-PO- (O-Am)₂) ou leurs mélanges (Am signifiant amidon).

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Un amidon préféré est un amidon ayant subi au moins une modification par voie chimique telle qu'au moins une estérification.

Selon l'invention, on peut aussi utiliser des amidons amphotères, ces amidons amphotères comprennent un ou plusieurs groupements anioniques et un ou plusieurs groupements cationiques. Les groupements anioniques et cationiques peuvent être liés au même site réactif de la molécule d'amidon ou à des sites réactifs différents, de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 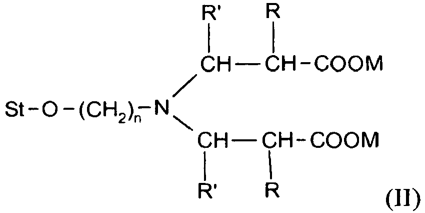 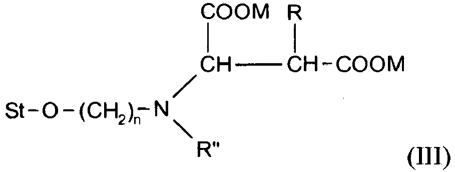 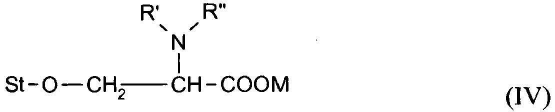 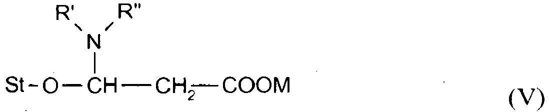 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (III) ou (IV). On utilise plus particulièrement les amidons modifiés par de l'acide 2-chloroéthyl aminodipropionique, c'est à dire les amidons de formule (III) ou (IV) dans lesquelles R, R', R" et M représentent un atome d'hydrogène et n est égal à 2. L'amidon amphotère préféré est un chloroéthylamidodipropionate d'amidon.

Comme indiqué précédemment, les dérivés de celluloses peuvent être notamment anioniques, amphotères ou non-ioniques.

Parmi ces dérivés, on distingue les éthers de celluloses, les esters de celluloses et les esters éthers de celluloses.

Parmi les esters de celluloses, on trouve les esters inorganiques de cellulose par exemple les nitrates, sulfates ou phosphates de cellulose, les esters organiques de cellulose par exemple les monoacétates, triacétates, amidopropionates, acétatebutyrates, acétatepropionates ou acétatetrimellitates de cellulose et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Parmi les éthers de cellulose non ioniques, on peut citer les alkylcelluloses telles que les méthylcelluloses et les éthylcelluloses (par exemple Ethocel standard 100 Premium de DOW CHEMICAL) ; les hydroxyalkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON) ; les celluloses mixtes hydroxyalkyl-alkylcelluloses telles que les hydroxypropyl-méthylcelluloses (par exemple Methocel E4M de DOW CHEMICAL), les hydroxyéthyl-méthylcelluloses, les hydroxyéthyl-éthylcellulose (par exemple Bermocoll E 481 FQ d'AKZO NOBEL) et les hydroxybutyl-méthylcelluloses.

Parmi les éthers de cellulose anioniques, on peut citer les carboxyalkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les polymères épaississants non associatifs sans motifs sucre utilisables, on peut citer les homopolymères ou copolymères d'acide acrylique ou méthacrylique réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide seuls ou leurs mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOL 980, 981, 954, 2984 et 5984 par la société NOVEON ou les produits vendus sous les noms SYNTHALEN M et SYNTHALEN K par la société 3 VSA.

Les polymères épaississants non associatifs peuvent être aussi des copolymères d'acide (méth)acryliques réticulés tels que le polymère vendu sous la dénomination AQUA SF1 par la société NOVEON.

Les polymères épaississants non associatifs peuvent être choisis parmi les homopolymères réticulés d'acide 2-acrylamido-2-méthylpropane sulfonique et leurs copolymères réticulés d'acrylamide.

En ce qui concerne ces homopolymères et copolymères, qui peuvent être partiellement ou totalement neutralisés, on peut citer les polymères comprenant de 90 à 99,9 % en poids, par rapport au poids total du polymère, de motifs de formule (j) suivante : 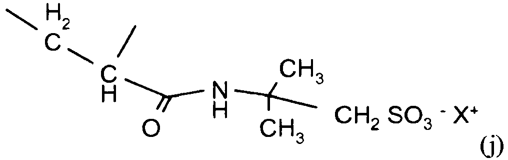 dans laquelle X+ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10 % en poids, par rapport au poids total du polymère, de motifs réticulants provenant d'au moins un monomère ayant au moins deux insaturations éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine, le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Pour plus de détail au sujet de ces polymères, on pourra se reporter au document EP 0 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2,798,053 et US 2,923,692 pour ce qui a trait à la description et à la préparation de tels composés.

Parmi les agents épaississants, on peut aussi citer les systèmes épaississants à base de polymères associatifs bien connus de l'homme de l'art et notamment de nature non ionique, anionique, cationique ou amphotère.

Il est rappelé que les polymères associatifs sont des polymères capables, dans un milieu aqueux, de s'associer réversiblement entre eux ou avec d'autres molécules.

Leur structure chimique comprend plus particulièrement au moins une zone hydrophile et au moins une zone hydrophobe.

Par groupement hydrophobe, on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (VI) suivante :

   CH₂ = CR'CH₂OBₙR (VI)
dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (VI) plus particulièrement préféré est un motif dans lequel R' désigne H, n est égal à 10, et R désigne un radical stéaryl (C₁₈).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention, les polymères formés à partir de 20 à 60 % en poids d'acide acrylique et/ou d'acide méthacrylique, de 5 à 60 % en poids de (méth)acrylates d'alkyles inférieurs, de 2 à 50 % en poids d'éther d'allyl à chaîne grasse de formule (VI), et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC80^{®} et SALCARE SC90^{®} qui sont des émulsions aqueuses à 30 °C d'un terpolymère réticulé d'acide méthacrylique, d'acrylate d'éthyle et de steareth-10-allyl éther (40/50/10) ;
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé ;

De préférence, ces polymères sont choisis parmi ceux dont le motif hydrophile de type acide carboxylique insaturé oléfinique correspond au monomère de formule (VII) suivante : 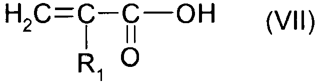 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (VIII) suivante : 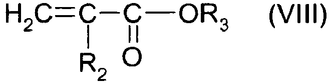 dans laquelle, R₂ désigne H ou CH₃ ou C₂H₅ (c'est-a-aire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₂₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US 3,915,921 et 4,509,949.

Parmi ce type de polymères associatifs anioniques, on utilisera plus particulièrement des polymères formés à partir d'un mélange de monomères comprenant :
(i) essentiellement de l'acide acrylique,
(ii) un ester de formule (VIII) décrite ci-dessus et dans laquelle R₂ désigne H ou CH₃, R₃ désignant un radical alkyle ayant de 12 à 22 atomes de carbone, et
(iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de à 60 à 95 % en poids d'acide acrylique (motif hydrophile), 4 à 40 % en poids d'acrylate d'alkyles en C₁₀-C₃₀ (motif hydrophobe), et 0 à 6 % en poids de monomère polymérisable réticulant, ou bien ceux constitués de 96 à 98 % en poids d'acide acrylique (motif hydrophile), 1 à 4 % en poids d'acrylate d'alkyles en C₁₀-C₃₀ (motif hydrophobe), et 0,1 à 0,6 % en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1^{®}, PEMULEN TR2^{®}, CARBOPOL 1382^{®}, et encore plus préférentiellement le PEMULEN TR1^{®}, et le produit vendu par la société S.E.P.P.I.C. sous la dénomination COATEX SX^{®}.

On peut aussi citer les polymères qui, outre les monomères de formule (VII) et de formule (VIII) contiennent un ou plusieurs autres monomères. Ce monomère additionnel peut être notamment un vinyllactame et en particulier la vinylpyrrolidone.

Comme exemple de polymère, on peut citer le terpolymère acide acrylique/méthacrylate de lauryle/vinylpyrrolidone commercialisé sous l'appellation Acrylidone LM par la Société ISP ;
- (III) les terpolymères d'anhydride maléique/α-oléfine en C₃₀-C₃₈/maléate d'alkyle tel que le produit (copolymère anhydride maléique/α-oléfine en C₁₀-C₃₈/maléate d'isopropyle) vendu sous le nom PERFORMA V 1608® par la société NEWPHASE TECHNOLOGIES ;
- (IV) les terpolymères acryliques comprenant :
   (a) environ 20 à 70 % en poids d'un acide carboxylique à insaturation α,β-monoéthylénique,
   (b) environ 20 à 80 % en poids d'un monomère à insaturation α,β-monoéthylénique non-tensio-actif différent de (a), et
   (c) environ 0,5 à 60 % en poids d'un mono-uréthane non-ionique qui est le produit de réaction d'un tensio-actif monohydrique avec un monoisocyanate à insaturation monoéthylénique,
      tels que ceux décrits dans la demande de brevet EP-A-0 173 109 et plus particulièrement celui décrit dans l'exemple 3, à savoir, un terpolymère acide méthacrylique/acrylate de méthyle/diméthyl métaisopropényl benzyl isocyanate d'alcool béhényle éthoxylé (40OE) en dispersion aqueuse à 25 %.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22^{®} vendu par la société ROHM et HAAS, qui est un terpolymère acide méthacrylique/acrylate d'éthyle/méthacrylate de stéaryle oxyalkyléné ; et
- (VI) Les polymères amphiphiles comportant au moins un monomère à insaturation éthylènique à groupement sulfonique, sous forme libre ou partiellement ou totalement neutralisée et comprenant au moins une partie hydrophobe. Ces polymères peuvent être réticulés ou non-réticulés. Ils sont de préférence réticulés.

Les monomères à insaturation éthylènique à groupement sulfonique sont choisis notamment parmi l'acide vinylsulfonique, l'acide styrènesulfonique, les acides (méth)acrytamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères de cette famille peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO 00/31154, faisant partie intégrante du contenu de la description. Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères préférés de cette famille sont choisis parmi les copolymères amphiphiles d'AMPS et d'au moins un monomère hydrophobe à insaturation éthylénique.

Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-0 750 899, le brevet US 5,089,578 et dans les publications de Yotaro Morishima suivantes :
- « Self-assembling amphiphilic polyelectrolytes and their nanostructures - Chinese Journal of Polymer Science Vol. 18, N° 40, (2000), 323-336 »;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N° 12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221».

Les monomères hydrophobes à insaturation éthylénique de ces copolymères particuliers sont choisis de préférence parmi les acrylates ou les acrylamides de formule (IX) suivante : 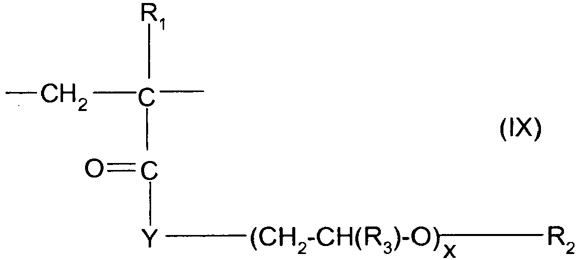 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins 8 et plus préférentiellement de 8 à 22 atomes de carbone et encore plus préférentiellement de 8 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₈-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃); le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (IX) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100, et plus préférentiellement de 3 à 50 et encore plus préférentiellement de 7 à 25, mieux de 8 à 25.

Parmi ces polymères, on peut citer :
- les copolymères réticulés ou non réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans la demande EP-A 0 750 899 ;
- les terpolymères comportant de 10 à 90 % en mole de motifs acrylamide, de 0,1 à 10 % en mole de motifs AMPS et de 5 à 80 % en mole de motifs n-(C₆-C₁₈)alkylacrylamide, tels que ceux décrits dans le brevet US-5,089,578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (X) suivante : 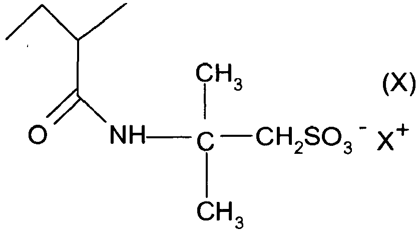 dans laquelle X⁺ est un proton, un cation de métal alcalin, un cation alcalino-terreux ou l'ion ammonium,
et de motifs de formule (XI) suivante : 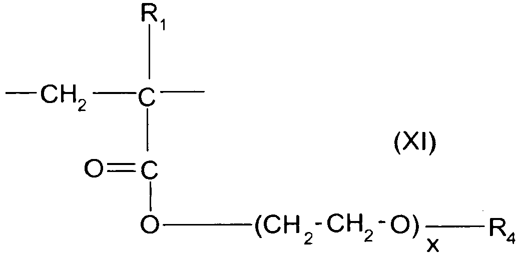 dans laquelle x désigne un nombre entier variant de 3 à 100, de préférence de 5 à 80, et plus préférentiellement de 7 à 25 ; R₁ a la même signification que celle indiquée ci-dessus dans la formule (IX) et R₄ désigne un alkyle linéaire ou ramifié en C₆-C₂₂ et plus préférentiellement en C₁₀-C₂₂, et mieux en C₁₂-C₁₈.

Les polymères particulièrement préférés sont ceux pour lesquels x = 25, R₁ désigne un méthyle et R₄ représente n-dodécyle ; ils sont décrits dans les articles de Morishima mentionnés ci-dessus.

La concentration molaire en % des motifs de formule (X) et des motifs de formule (XI) variera en fonction de l'application cosmétique souhaitée et des propriétés rhéologiques de la formulation recherchées. Elle varie de préférence de 70 à 99 % en moles de motifs AMPS et de 1 à 30 % en moles de motifs de formule (XI) par rapport au copolymère et plus particulièrement de 70 à 90 % en moles de motifs AMPS et de 10 à 30 % en moles de motifs de formule (XI).

Les polymères pour lesquels X+ désigne l'ion sodium ou l'ion ammonium sont plus particulièrement préférés.

Parmi les polymères associatifs de type cationique, on peut citer :
- (I) les polyuréthanes associatifs cationiques dont la famille a été décrite par la demanderesse dans la demande de brevet français n° 0009609; elle peut être représentée par la formule générale (XII) suivante :

   R-X-(P)ₙ-[L-(Y)ₘ]ᵣ-L'-(P')ₚ-X'-R' (XII)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 est 50 et en particulier entre 1 et 25,
   n, m, et p valent chacun indépendamment des autres entre 0 et 1000 ;
   la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (XII) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (XII) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (XII) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 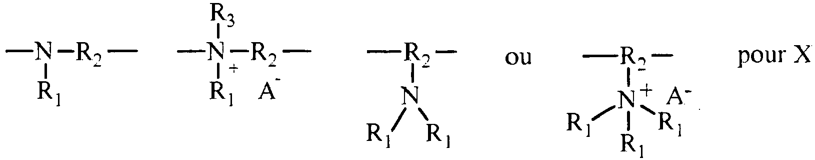 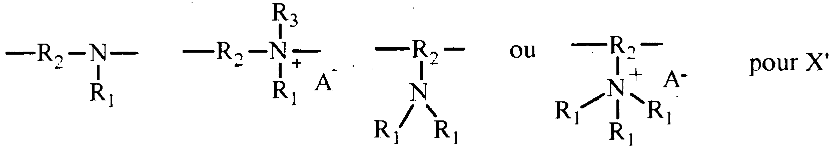 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 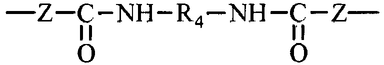 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 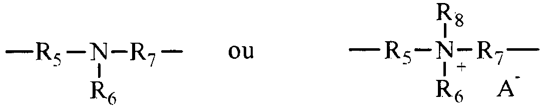 ou 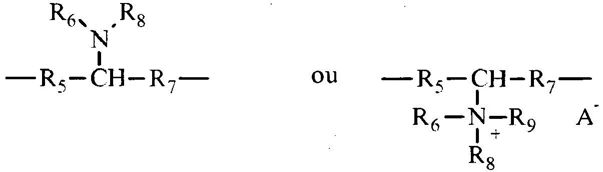 ou 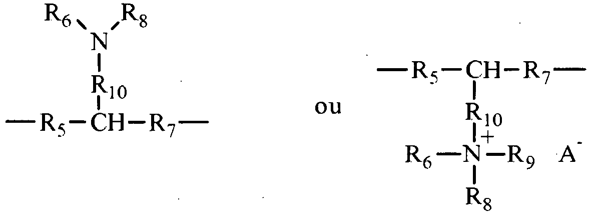 dans lesquelles :
R₅ et R₇ ont les mêmes significations que R₂ défini précédemment ;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (XII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (XII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (XII) est un diisocyanate correspondant à la formule :

O=C=N-R₄-N=C=O

dans laquelle R₄ est défini plus haut.

A titre d'exemple, on peut citer le méthylènediphényl-düsocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (XII) est un composé hydrophobe destiné à former les groupes hydrophobes terminaux du polymère de formule (XII).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (XII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (XII) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) le ou les polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs.

En particulier, parmi ces polymères cationiques, on peut notamment citer le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
- un méthacrylate de di(alkyl en C1-C4) amino(alkyle en C1-C6),
- un ou plusieurs esters d'alkyle en C1-C30 et de l'acide (méth)acrylique,
- un méthacrylate d'alkyle en C10-C30 polyéthoxylé (20-25 moles de motif oxyde d'éthylène),
- un allyl éther de polyéthylèneglycol/polypropylèneglycol 30/5,
- un méthacrylate d'hydroxy(alkyle en C2-C6), et
- un diméthacrylate d'éthylèneglycol.
- (III) les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) vendus par la société AMERCHOL, les produits CRODACEL QM, CRODACEL QL (alkyle en C₁₂) et CRODACEL QS (alkyle en C₁₈) vendus par la société CRODA et le produit SOFTCAT SL 100 vendu par la société AMERCHOL.
- (IV) les polymères polyvinyllactames cationiques comprenant :
- a) au moins un monomère de type vinyl lactame ou alkylvinyllactame;
- b) au moins un monomère de structures (XIII) ou (XIV) suivantes : 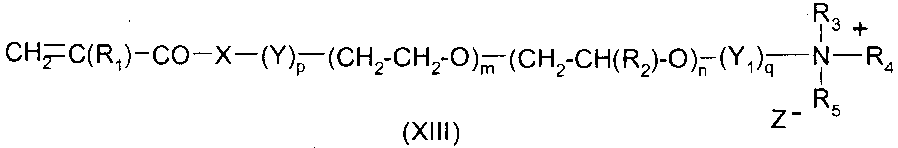 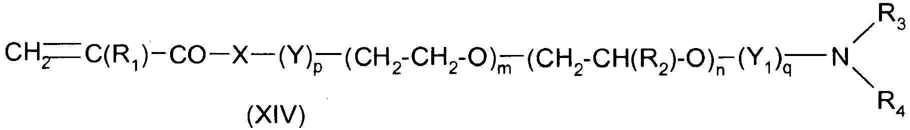
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₂₀ ou un radical de formule (XV) :

   -(Y₂)ᵣ-(CH₂-CH(R₇)-O)ₓ-R₈ (XV)
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m et n sont égaux à zéro, alors un de p ou q est égal à 0.

Les polymères poly(vinyllactame) cationiques selon l'invention peuvent être réticulés ou non réticulés et peuvent aussi être des polymères blocs.

De préférence le contre ion Z⁻ des monomères de formule (XIII) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (XII) pour laquelle, encore plus préférentiellement, m et n sont égaux à zéro.

Le monomère vinyl lactame ou alkylvinyllactame est de préférence un composé de structure (XVI) : 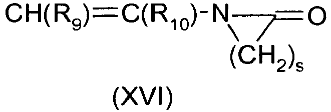 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (XVI) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a) -un monomère de formule (XVI),
b) -un monomère de formule (XIII) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c) -un monomère de formule (XIV) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95 % de monomère (a), 0,1 à 55 % de monomère (c) et 0,25 à 50 % de monomère (b).

De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone/diméthylaminopropylméthacrylamide/tosylate de dodécyldiméthylméthacrylamidopropylammonium, les terpolymères vinylpyrrolidone/diméthylaminopropylméthacrylamide/tosylate de cocoyldiméthylméthacrylamidopropylammonium, les terpolymères vinylpyrrolidone/diméthylaminopropylméthacrylamide/tosylate ou chlorure de lauryldiméthylméthacrylamidopropylammonium.

Les polymères associatifs amphotères sont choisis de préférence parmi ceux comportant au moins un motif cationique non cyclique. Plus particulièrement encore, on préfère ceux préparés à partir ou comprenant 1 à 20 % en moles de monomère comportant une chaîne grasse, et de préférence 1,5 à 15 % en moles et plus particulièrement encore 1,5 à 6 % en moles par rapport au nombre total de moles de monomères.

Les polymères associatifs amphotères préférés selon l'invention comprennent, ou sont préparés en copolymérisant :
1) au moins un monomère de formule (XVII) ou (XVIII) : 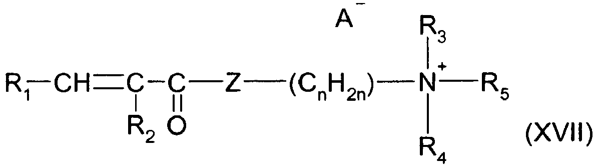 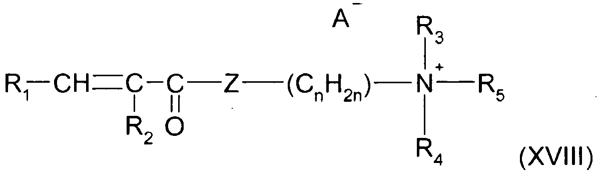 dans lesquelles, R₁ et R₂, identiques ou différents, représentent un atome d'hydrogène ou un radical méthyle, R₃, R₄ et R₅, identiques ou différents, représentent un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (XIX) 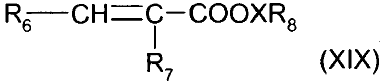 dans laquelle, R₆ et R₇, identiques ou différents, représentent un atome d'hydrogène ou un radical méthyle;
   et
3) au moins un monomère de formule (XX) : 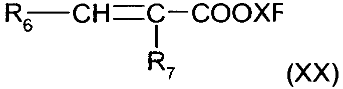
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (XVII), (XVIII) ou (XX) comportant au moins une chaîne grasse.

Les monomères de formule (XVII) et (XVIII) sont choisis, de préférence, dans le groupe constitué par :
- le diméthylaminoéthyl méthacrylate, le diméthylaminoéthyl acrylate,
- le diéthylaminoéthyl méthacrylate, le diéthylaminoéthyl acrylate,
- le diméthylaminopropylméthacrylate, le diméthylaminopropyl acrylate,
- le diméthylaminopropyl méthacrylamide, le diméthylaminopropyl acrylamide,
ces monomères étant éventuellement quaternisés, par exemple par un halogénure d'alkyle en C₁-C₄ ou un sulfate de dialkyle en C₁-C₄.

Plus particulièrement, le monomère de formule (XVII) est choisi parmi le chlorure d'acrylamidopropyl triméthyl ammonium et le chlorure de méthacrylamidopropyl triméthyl ammonium.

Les monomères de formule (XIX) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XIX) est l'acide acrylique.

Les monomères de formule (XX) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères de cette famille comprennent de préférence de 1 à 10 % moles du monomère comportant une chaîne grasse (monomère de formule (XVII), (XVIII) ou (XX), et de préférence de 1,5 à 6 % moles.

Les polymères associatifs amphotères de cette famille peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO/9844012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (a) les copolymères de vinyl pyrrolidone et de monomères hydrophobes à chaîne grasse dont on peut citer à titre d'exemple :
   - les produits ANTARON V216^{®} ou GANTEX V216^{®} (copolymère vinylpyrrolidone / hexadécène) vendu par la société I.S.P.
   - les produits ANTARON V220^{®} ou GANTEX V220^{®} (copolymère vinylpyrrolidone / eicosène) vendu par la société I.S.P.
- (b) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208^{®}.
- (c) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (d) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (e) les polymères à squelette aminoplaste éther possédant au moins une chaîne grasse, tels que les composés PURE THIX^{®} proposés par la société SUD-CHEMIE.
- (f) les celluloses ou leurs dérivés, modifiés par des groupements comportant au moins une chaîne grasse tels que des groupes alkyls, arylalkyls, alkylaryls ou leurs mélanges où les groupes alkyls sont en C₈ et en particulier :
   - les alkylhydroxyéthylcelluloses non-ioniques telles que les produits NATROSOL PLUS GRADE 330 CS et POLYSURF 67 (alkyle en C₁₆) vendus par la société AQUALON
   - les nonoxynylhydroxyéthylcelluloses non-ioniques telles que le produit AMERCELL HM-1500 vendu par la société AMERCHOL ;
   - les alkylcelluloses non-ioniques telles que le produit BERMOCOLL EHM 100 vendu par la société BEROL NOBEL ;
- (g) les dérivés de guar associatifs comme les hydroxypropylguars modifiés par une chaîne grasse tel que le produit ESAFLOR HM 22 (modifié par une chaîne alkyle en C₂₂) vendu par la société LAMBERTI ; le produit MIRACARE XC 95-3 (modifié par une chaîne alkyle en C₁₄) et le produit RE 205-146 (modifié par une chaîne alkyle en C₂₀) vendus par RHODIA CHIMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205^{®} à fonction urée vendu par la société RHEOX ou encore les Rhéolates^{®} 208, 204 ou 212, ainsi que l'Acrysol RM 184^{®}.

On peut également citer le produit ELFACOS T210^{®} à chaîne alkyle en C₁₂-C₁₄ et le produit ELFACOS T212^{®} à chaîne alkyle en C₁₈ de chez AKZO.

Le produit DW 1206B^{®} de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le RHEOLATE^{®} 255, le RHEOLATE^{®} 278 et le RHEOLATE^{®} 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations ACULYN 46^{®} et ACULYN 44^{®} [l'ACULYN 46^{®} est un polycondensat de polyéthylèneglycol à 150 ou 180 moles d'oxyde d'éthylène, d'alcool stéarylique et de méthylène bis(4-cyclohexyl-isocyanate) (SMDI), à 15 % en poids dans une matrice de maltodextrine (4 %) et d'eau (81 %); l'ACULYN 44^{®} est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %)].

Encore plus préférentiellement le ou les polymères épaississants de l'invention sont choisis parmi les polymères à motifs sucre associatifs ou non, les polymères anioniques acryliques ou méthacryliques associatifs ou non, et les polyuréthanes associatifs ou non.

Les polymères conditionneurs préférés sont choisis de préférence parmi les polymères conditionneurs cationiques ou amphotères et de préférence cationiques.

Parmi les polymères conditionneurs susceptibles d'être utilisés, on préfère mettre en oeuvre les dérivés d'éther de cellulose quaternaires tels que les produits vendus sous la dénomination JR 400 ou Quetrisoft LM 200 par la société Amerchol, les cyclopolymères cationiques, en particulier les homopolymères ou copolymères de chlorure de diméthyldiallylammonium, tels que ceux vendus sous les dénominations Merquat^{®} 100, Merquat^{®} 550 et Merqual^{®} S par la société Nalco, les gommes de guar modifiées cationiques telles que le Jaguar C13S, les polymères quaternaires de vinylpyrrolidone et de vinylimidazole, les homopolymères de sels de méthacrylate d'éthyltriméthylammonium (SALCARE SC95 ou 96) et leurs mélanges.

Tout particulièrement les polymères non siliconés de l'invention sont choisis parmi les polymères à motifs sucre associatifs ou non, mieux parmi les polysaccharides de type hydroxyéthylcellulose et hydroxypropyl guar.

Le ou les polymères non siliconés peuvent notamment être présents en une teneur allant de 0,01 à 20 % en poids, de préférence de 0,1 à 10 % en poids, mieux de 0,2 à 5 % en poids, par rapport au poids total de la composition.

Le rapport pondéral de la quantité de particules de pierre ponce à la quantité de polymères non siliconés varie de préférence de 1 à 200, encore plus préférentiellement de 3 à 100, mieux de 5 à 50.

### Autres composants de la composition capillaire

Outre les particules de pierre ponce, la ou les silicones à groupements ammonium quaternaire et le ou les polymères non siliconés conformes à l'invention, la composition capillaire peut comprendre un ou plusieurs additifs.

La composition capillaire peut ainsi comprendre un ou plusieurs tensioactifs additionnels tels que des tensioactifs anioniques, amphotères, zwittérioniques ou non ioniques.

Le ou les tensioactifs additionnels sont de préférence choisis parmi les tensioactifs non ioniques.

Les agents tensioactifs non-ioniques sont des composés bien connus en soi (voir notamment à cet égard « Handbook of Surfactants » par M.R. Porter, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Le ou les tensioactifs non ioniques peuvent être présents dans la composition capillaire dans des concentrations allant de 0,1 à 25 % en poids, de préférence de 1 à 20 % en poids par rapport au poids total de la composition.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits vendus sous la dénomination MIRANOL, tel que décrits dans les brevets US 2,528,378 et US 2,781,354 et classés dans le dictionnaire CTFA, 3ème édition, 1982, sous les dénominations amphocarboxyglycinates et amphocarboxypropionates de structures respectives :

R₂ -CONHCH₂CH₂-N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂' -CONHCH₂CH₂-N(B)(C)

dans laquelle :
B représente -CH₂CH₂OX', C représente -(CH₂)_{Z}-Y', avec z = 1 ou 2,
X' désigne le groupement -CH₂CH₂-COOH ou un atome d'hydrogène,
Y' désigne -COOH ou le radical -CH₂CHOH-SO₃H,
R₂' désigne un radical alkyle d'un acide R₉-COOH présent dans l'huile de coprah ou dans l'huile de lin hydrolysée, un radical alkyle, notamment en C₇, C₉, C₁₁, ou C₁₃, un radical alkyle en C₁₇ et sa forme iso, un radical C₁₇ insaturé.

Ces composés sont classés dans le dictionnaire CTFA, 5ème édition, 1993, sous les dénominations disodium cocoamphodiacetate, disodium lauroamphodiacetate, disodium caprylamphodiacetate, disodium capryloamphodiacetate, disodium ocoamphodipropionate, disodium lauroamphodipropionate, disodium caprylampho-dipropionate, disodium capryloamphodipropionate, lauroamphodipropionic acid, cocoamphodipropionic acid, disodium cocoamphocarboxyl ethyl hydroxypropyl sulfonate.

A titre d'exemple, on peut citer le cocoamphodiacetate commercialisé sous la dénomination commerciale MIRANOL^{®} C2M concentré par la société Rhodia Chimie.

Pour ce qui concerne les tensioactifs anioniques, on peut citer de manière non limitative les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfne-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucosides citrates, les alkylpolyglycosides tartrates et les alkylpolyglycosides sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Les tensioactifs anioniques éventuellement présents sont de préférence des tensioactifs anioniques doux.

En ce qui concerne les tensioactifs anioniques doux, on peut citer notamment les composés suivants et leurs sels, ainsi que leurs mélanges :
- les acides alkyl éther carboxyliques polyoxyalkylénés ;
- les acides alkylaryl éther carboxyliques polyoxyalkylénés :
- les acides alkylamido éther carboxyliques polyoxyalkylénés en particulier ceux comportant 2 à 50 groupements oxyde d'éthylène ;
- les acides d'alkyl D galactoside uroniques ;
- les acylsarcosinates, les acylglutamates ; et
- les esters d'alkylpolyglycosides carboxyliques.

Tout particulièrement, on peut utiliser des acides alkyl éther carboxyliques polyoxyalkylénés comme par exemple l'acide lauryl ether carboxylique (4,5 OE) commercialisé par exemple sous la dénomination AKYPO RLM 45 CA de KAO.

Si de tels tensioactifs anioniques ou amphotères sont présents, alors leur teneur va de 0,1 à 20 % en poids, par rapport au poids total de la composition capillaire, plus particulièrement de 1 à 10 % en poids, par rapport au poids total de la composition.

De préférence, la composition capillaire ne contient pas de tensioactif détergent anionique de type sulfate (alkyl sulfate ou alkyl éther sulfate, alkyl amido éther sulfate). Et si elle en contient, sa teneur est telle que le rapport pondéré : tensioactif détergent anionique de type alkyl sulfate ou alkyl éther sulfate/somme des autres tensioactifs non cationiques, soit de préférence inférieur ou égal à 1, et plus particulièrement inférieur ou égal à 0,75 et encore plus préférentiellement inférieur ou égal à 0,5.

Les compositions de l'invention peuvent aussi contenir un ou plusieurs tensioactifs cationiques.

A titre de tensioactifs cationiques, on peut par exemple citer ceux comportant au moins une fonction ester.

La composition capillaire peut de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les colorants directs ou d'oxydation, les parfums, les peptisants, les conservateurs, les vitamines, les agents antipelliculaires, les agents antiséborrhéïques, les agents antichute des cheveux, les agents épaississants non polymériques tels que les amides grasses, les éthers gras, les alcools gras, les silices, les argiles, etc.

Les adjuvants cités ci-dessus sont en général présents en quantité comprise, pour chacun d'eux, entre 0,01 et 20 % en poids par rapport au poids de la composition.

La composition capillaire peut comprendre un ou plusieurs agents conditionneurs non polymériques ou siliconés non quaternaires additionnels.

Lorsque la composition capillaire contient au moins un agent conditionneur additionnel, celui-ci peut être choisi parmi les huiles de synthèse telles que les poly-α-oléfines, les huiles fluorées, les cires fluorées, les gommes fluorées, les esters d'acides carboxyliques, les tensioactifs cationiques des silicones distinctes des silicones à groupements ammonium quaternaire requises selon l'invention, les huiles minérales, végétales ou animales, les céramides, les pseudocéramides, les agents épaississants non polymériques et leurs mélanges.

Parmi les tensioactifs cationiques, on peut citer en particulier (liste non limitative) : les sels d'amines grasses primaires, secondaires ou tertiaires, éventuellement polyoxyalkylénées ; les dérivés d'imidazoline ; les sels d'ammonium quaternaire et en particulier les sels de trialkylammonium comportant au moins une chaîne grasse ayant de 10 à 30 atomes de carbone et notamment les sels de cétyltriméthymammonium ou de béhényltriméthylammonium ; les tensioactifs cationiques comportant au moins une fonction ester, et par exemple un sel de diacyloxydialkyl-hydroxyalkylammonium ou un sel de diacyloxytrialkylammonium et en particulier le dipalmitoyléthyl hydroxyéthylammonium méthosulfate, le dicétéaroyléthylhydroxyéthyl méthylammonium méthosulfate, le chlorure de distéaroyléthyldiméthylammonium ou le chlorure de distéaroyldiéthylméthylammonium, et de préférence le dipalmitoyléthyl hydroxyéthylammonium méthosulfate.

La teneur en agents conditionneurs additionnels non polymériques ou siliconés non quaternaires dans la composition capillaire peut aller de 0,001 % à 10 % en poids, de préférence de 0,005 % à 5 % en poids, et encore plus préférentiellement de 0,01 % à 3 % en poids par rapport au poids total de la composition finale.

Le milieu aqueux acceptable pour les cheveux peut comporter de l'eau ou un mélange d'eau et un ou plusieurs solvants organiques acceptables sur le plan cosmétique.

La teneur en eau de la composition est de préférence supérieure ou égale à 50 % en poids par rapport au poids total de la composition.

A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.

Le ou les solvants organiques peuvent être présents dans des proportions par exemple comprises entre 1 et 40 % en poids environ par rapport au poids total de la composition capillaire, et encore plus préférentiellement entre 5 et 30 % en poids environ.

### Forme galénique

La composition capillaire selon l'invention peut se présenter sous différentes formes galéniques telles qu'une lotion, un shampoing, un gel, une crème, une cire. La composition capillaire peut être conditionnée dans tout type de récipient avec applicateur ou non. Le récipient peut contenir une bille ou un organe permettant notamment d'homogénéiser la composition capillaire avant application de celle-ci sur les cheveux. Le récipient contenant la composition capillaire peut avoir une contenance supérieure ou égale à 15 ml, notamment supérieure ou égale à 50 ml, voire à 100 ml, notamment supérieure ou égale à 150 ml, par exemple comprise entre 15 ml et 500 ml.

La composition capillaire peut imprégner une lingette.

La composition capillaire peut être introduite dans un courant de fluide vecteur, et être projetée sur les cheveux.

Dans un mode de réalisation particulier, la composition capillaire peut être contenue dans un réservoir d'un peigne ou d'une brosse présentant par exemple des orifices à la base des dents ou poils permettant d'amener le produit et de l'appliquer sur les cheveux.

### Procédé de traitement

L'invention concerne également un procédé de traitement des cheveux consistant à appliquer sur les cheveux au moins une composition capillaire telle que définie précédemment, puis éventuellement à effectuer un rinçage.

Le traitement, par exemple l'abrasion, des cheveux peut avoir lieu immédiatement après l'application de la composition capillaire sur les cheveux, par exemple dans un délai inférieur à une heure, notamment inférieur à 30 min, voire à 10 min après l'application de la composition capillaire sur les cheveux. Un rinçage des cheveux, lorsqu'il a lieu, peut intervenir rapidement après traitement, par exemple abrasion, des cheveux. Le temps entre l'application de la composition et le rinçage peut par exemple être inférieur à 4h, notamment inférieur à 1h, par exemple inférieur à 20 min.

L'étape de traitement, par exemple d'abrasion, des cheveux peut être effectuée en frottant les cheveux avec les mains nues ou en interposant au moins une surface entre les mains et les cheveux chargés de composition capillaire, par exemple une lingette ou serviette, un gant ou autre. Le traitement peut être effectué autrement qu'à l'aide d'un peigne ou d'une brosse.

Le traitement, par exemple l'abrasion, des cheveux peut par exemple être réalisé à l'aide d'au moins une surface mise en mouvement, en vibration ou en rotation notamment, par un système mécanisé.

Le traitement à l'aide de la composition capillaire peut par exemple être effectué, à la main par exemple, mèche par mèche, par exemple en deux mouvements : un premier mouvement dit « tangentiel », c'est-à-dire sensiblement le long des cheveux, par exemple de la racine vers la pointe, et un deuxième mouvement dit « de cisaillement », c'est-à-dire sensiblement orthogonalement aux cheveux, dans une direction transversale à ceux-ci. Les deux mouvements peuvent être répétés plusieurs fois, par exemple au moins cinq fois, par exemple dix fois. En variante, un seul des mouvements précités peut être effectué et éventuellement répété plusieurs fois.

La durée de traitement, par exemple d'abrasion, au moyen de la composition conforme à l'invention peut dépendre par exemple de l'intensité recherchée de l'abrasion et de l'état du cheveu.

Le procédé selon l'invention peut comporter deux étapes de traitement, par exemple d'abrasion, à l'aide de deux compositions capillaires successivement appliquées, chacune comportant des particules de pierre ponce conformes à l'invention, les diamètres moyens en volume ou la dureté des particules de pierre ponce conformes à l'invention des deux compositions capillaires étant différentes.

Un flacon unique peut contenir les deux compositions capillaires conditionnées séparément, ou deux flacons différents peuvent contenir chacun une composition capillaire.

Un autre mode de conditionnement peut consister en un flacon contenant une composition capillaire de base dépourvue de particules de pierre ponce conformes à l'invention, lesquelles sont conditionnées à part, par exemple dans au moins deux compartiments séparés, en fonction de leur granulométrie ou de leur dureté. Dans ce dernier cas, l'utilisateur choisit avant le traitement, par exemple l'abrasion, les particules de pierre ponce conformes à l'invention à mélanger à la composition de base pour former la première composition capillaire, le mélange étant effectué par l'utilisateur ou au sein du flacon. L'utilisateur peut, après le premier traitement, par exemple la première abrasion, effectuer un deuxième traitement, par exemple une deuxième abrasion, en mélangeant cette fois-ci d'autres particules de pierre ponce conformes à l'invention à la composition capillaire de base pour former la deuxième composition capillaire. Un rinçage peut être effectué entre et/ou après les deux traitements.

Le procédé de traitement peut être mis en oeuvre après une étape de caractérisation du cheveu, par exemple à l'aide d'un examen visuel à l'oeil nu ou sous un dispositif d'agrandissement ou par voie instrumentale, par exemple en enregistrant le son produit par le déplacement d'un peigne dans les cheveux à l'aide d'un sonomètre ou en déterminant optiquement la brillance des cheveux. La caractérisation du cheveu peut encore faire intervenir un réactif chimique appliqué sur un échantillon de cheveu.

Le procédé selon l'invention peut comporter l'étape consistant à peigner et/ou rincer les cheveux après application de la composition capillaire. Le rinçage peut être effectué à l'eau.

Le procédé selon l'invention peut également comporter l'étape consistant à chauffer les cheveux avant ou après mise en contact avec la composition capillaire, par exemple à une température comprise entre 40 °C et 250 °C, notamment entre 60 °C et 220 °C. On peut par exemple chauffer les cheveux après le traitement, par exemple l'abrasion, de manière à les mettre en forme, par exemple en effectuant un brushing. Le chauffage des cheveux peut par exemple être effectué au moyen d'un fer, d'un mélange eau liquide/vapeur d'eau ou au moyen d'un casque chauffant.

Les cheveux peuvent être séchés totalement ou partiellement.

### Application d'un produit de traitement

Le procédé selon l'invention peut comporter en outre l'étape consistant à appliquer, par exemple avant ou après traitement à l'aide de la composition capillaire selon l'invention, un autre produit de traitement sur les cheveux. Le produit de traitement peut être par exemple un produit cosmétique, notamment un conditionneur, une permanente, un défrisant, un produit de décoloration ou de coloration des cheveux.

Le produit de traitement peut être par exemple choisi parmi les suivants, cette liste n'étant pas limitative:
- les produits visant à modifier les propriétés mécaniques des cheveux, notamment comportant un réducteur, tel que l'acide thioglycolique et ses dérivés, la cystéine, le sulfite, la soude, le carbonate de guanidine, la trihydroxyméthyl phosphine, ou un oxydant, tel que H₂O₂, le persulfate ;
- les produits émollients ou de pénétration, comportant par exemple un solvant, un glycol, un plastifiant, ou un tensioactif cationique, anionique ou amphotère ;
- les produits modifiant les propriétés de la surface du cheveu, comportant notamment une silicone, une silicone aminée réactive, un polymère adhésif, un lubrifiant non siliconé comportant des corps gras choisis parmi les huiles végétales, les huiles minérales, les huiles de synthèse, les cires notamment des alcools gras ou des esters gras ;
- les produits restructurant l'intérieur du cheveu comportant par exemple un ionène, une protéine, un hydroxyacide ou un composé réactif, notamment un générateur de formol, un silane ; et
- les colorants directs ou d'oxydation.

Le produit de traitement peut être appliqué préalablement au traitement, par exemple de type abrasion, et peut contribuer à protéger les cheveux pendant celui-ci, afin d'éviter notamment une abrasion excessive.

Le produit de traitement est de préférence appliqué après application de la composition conforme à l'invention.

Ainsi, selon un mode de réalisation, le procédé conforme à l'invention peut comporter en outre l'étape consistant à soumettre les cheveux à un post-traitement, après traitement à l'aide d'une composition capillaire telle que définie précédemment, le post-traitement étant choisi parmi l'application d'un conditionneur, d'une permanente, d'un défrisant, d'un produit de coloration ou de décoloration des cheveux.

### Kits de traitement

L'invention a encore pour objet, indépendamment ou en combinaison avec ce qui précède, un kit de traitement des cheveux comportant :
- une composition capillaire comportant au moins des particules de pierre ponce, une ou plusieurs silicones à groupements ammonium quaternaire et un ou plusieurs polymères non siliconés, et
- un support comportant des instructions pour l'emploi de la composition capillaire sur les cheveux, par exemple en vue de réaliser une abrasion des cheveux.

La composition capillaire est telle que décrite plus haut.

Le kit peut comporter en outre une composition de post-traitement des cheveux. La composition de post-traitement peut être choisie parmi un conditionneur, une permanente, un défrisant, un produit de coloration ou de décoloration des cheveux. Le produit de post-traitement peut par exemple être choisi parmi ceux décrits plus haut.

### EXEMPLE :

### (les proportions sont pondérales par rapport au poids total de la composition)

### Exemple comparatif 1 - Mise en évidence du pouvoir lissant

Les compositions de traitement suivantes ont été élaborées

| Composition | A | B |
|---|---|---|
| Caprylyl glycol | 0,5 | 0,5 |
| Cétrimonium chloride | 0,5 | 0,5 |
| Pumice (Pierre Ponce 0 ½ D d'EYRAUD) | 18 | 0 |
| Quaternium-80 (ABIL QUAT 3272 de GOLDSCHMIDT) | 0,2 | 0,2 |
| Glycéryl stéarate | 1,2 | 1,2 |
| Hydroxypropyl guar (JAGUAR HP 105 de RHODIA) | 0,1 | 0,1 |
| Glycérine | 2 | 2 |
| Propylène glycol | 0,5 | 0,5 |
| Cétéaryl alcohol | 1 | 1 |
| Hydroxyéthylcellulose (NATROSOL 250 HHR de AQUALON) | 0,8 | 0,8 |
| Conservateurs | 0,2 | 0,2 |
| Dipalmitoyléthyl hydroxyéthylmonium méthosulfate/Cétéaryl alcohol (30/70 en poids) (DEHYQUART F30 de COGNIS) | 4,3 | 4,3 |
| Eau | Qsp 100 | Qsp 100 |

L'étude est réalisée sur un panel de 20 femmes : 10 femmes aux cheveux longs naturels et 10 femmes aux cheveux longs sensibilisés.

10 à 15 g de chacun de ces traitements sont appliqués par demi-tête.

L'application se fait mèche par mèche en deux mouvements : un premier mouvement dit « tangentiel » et un deuxième mouvement dit « de cisaillement ». Les deux mouvements sont répétés 10 fois. Les cheveux sont peignés, puis rincés et enfin séchés par brushing.

### Analyse sensorielle

Les résultats montrent que pour 7 femmes aux cheveux naturels sur 10 et pour 9 femmes aux cheveux sensibilisés sur 10, le côté traité par le procédé de l'invention avec la composition A est plus lisse, plus souple et plus homogène que le côté traité avec la composition B. L'invention permet de réduire la masse et de maîtriser le volume des cheveux et d'améliorer la discipline de la chevelure. Les effets sont d'autant plus remarquables que les cheveux sont sensibilisés et épais.

Après 5 shampooings, le lissage des cheveux est conservé ainsi que la maîtrise du volume quel que soit le degré de sensibilité des cheveux (naturels à très sensibilisés).

### Exemple 2 :

On a préparé la composition suivante :

| | |
|---|---|
| Caprylyl glycol | 0,5 |
| Cétrimonium chloride | 0,5 |
| Pumice (Pierre Ponce 2B D d'EYRAUD de taille allant de 100 à 500 µm) | 25 |
| Quaternium-80 (ABIL QUAT 3272 de GOLDSCHMIDT) | 0,2 |
| Glycéryl stéarate | 1,2 |
| Hydroxypropyl guar (JAGUAR HP 105 de RHODIA) | 0,1 |
| Glycérine | 2 |
| Propylène glycol | 0,5 |
| Cétéaryl alcohol | 1 |
| Hydroxyéthylcellulose (NATROSOL 250 HHR de AQUALON) | 0,8 |
| Conservateurs | 0,2 |
| Dipalmitoyléthyl hydroxyéthylmonium méthosulfate /Cétéaryl alcohol (30/70 en poids) (DEHYQUART F30 de COGNIS) | 10 |
| Eau | Qsp 100 |

### Exemple 3 :

On a préparé la composition suivante :

| | |
|---|---|
| Caprylyl glycol | 0,1 |
| Cétrimonium chloride | 0,5 |
| Pumice (Pierre Ponce 0 ½ D d'EYRAUD) | 15 |
| Quaternium-80 (ABIL QUAT 3474 de GOLDSCHMIDT) | 0,1 |
| Glycéryl stéarate | 1,2 |
| Hydroxypropyl guar (JAGUAR HP 105 de RHODIA) | 0,1 |
| Glycérine | 2 |
| Propylène glycol | 0,5 |
| Cétéaryl alcohol | 1 |
| Hydroxyéthylcellulose (NATROSOL 250 HHR de AQUALON) | 0,8 |
| Conservateurs | 0,2 |
| Dipalmitoyléthyl hydroxyéthylmonium méthosulfate /Cétéaryl alcohol (30/70 en poids) (DEHYQUART F30 de COGNIS) | 4,3 |
| Eau | Qsp 100 |

L'invention n'est pas limitée aux exemples décrits.

L'expression « comportant un » doit être comprise comme étant synonyme de « comportant au moins un » et « compris entre » ou « allant de » s'entendent bornes incluses, sauf si le contraire est spécifié.

## Revendications

1. Composition capillaire comprenant au moins des particules de pierre ponce, une ou plusieurs silicones à groupements ammonium quaternaire et un ou plusieurs polymères non siliconés.

2. Composition selon la revendication 1, les particules de pierre ponce ayant un diamètre moyen en volume inférieur ou égal à 500 µm, de préférence compris entre 50 et 500 µm, mieux inférieur ou égal à 300 µm, par exemple compris entre 100 et 300 µm.

3. Composition selon l'une quelconque des revendications précédentes, dans laquelle les particules de pierre ponce sont présentes en une teneur allant de 0,1 % à 35 % en poids, notamment de 5 % à 30 % en poids, en particulier de 10 % à 25 % en poids, par exemple de 15 % à 20 % en poids, mieux de 18 % à 20% en poids par rapport au poids total de la composition.

4. Composition selon l'une quelconque des revendications précédentes, dans laquelle les particules de pierre ponce ont une dureté allant de 3 à 10 Mohs, voire supérieure ou égale à 4, par exemple supérieure ou égale à 5, et en particulier allant de 5 à 5,5 sur l'échelle de Mohs.

5. Composition selon l'une quelconque des revendications précédentes, dans laquelle ladite silicone à groupements ammonium quaternaire est choisie parmi les composés correspondants aux formules générales suivantes : 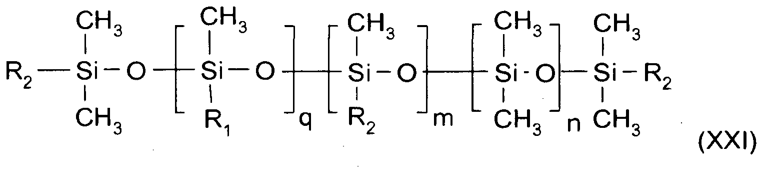 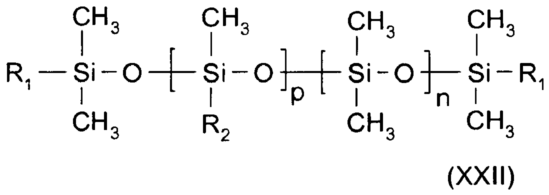 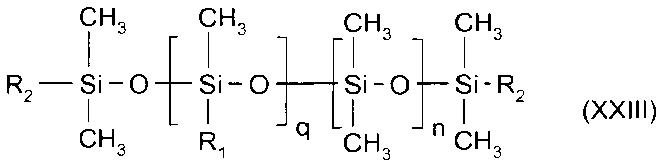 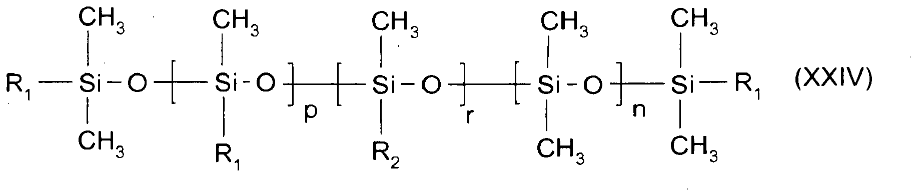 dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-(PO₃H)_{d} -R₅ ou -C_{c}H_{2c}-O-(C₄H₈O)ₐ-(PO₃H)_{d} -R₅ ;
R₅, identique ou différent, est choisi parmi les groupes de formule suivante : 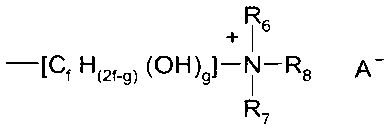
- les radicaux R₈ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZCOR₉; et
- R₆, R₇ et R₉, identiques ou différents, représentent des radicaux alkyle en C₁₋₂₂ ou alcényle en C₂₋₂₂, linéaires ou ramifiés, portant éventuellement un ou plusieurs groupements OH, ou R₇ peut former avec une partie de R₈ un hétérocycle (cycle avec au moins un hétéroatome tel que par exemple N, O, P), l'hétérocycle est notamment une imidazoline,
- m varie de 0 à 20 ;
- n varie de 0 à 500 ;
- p varie de 1 à 50 ;
- q varie de 0 à 20 ;
- r varie de 1 à 20 ;
- a varie de 0 à 50 ;
- b varie de 0 à 50 ;
- c varie de 0 à 4 ;
- d désigne 0 où 1 ;
- f varie de 0 à 4 ;
- g varie de 0 à 2, de préférence est égal à 1 ; et
- h varie de 1 à 4, de préférence est égal à 3,
Z représente un atome d'oxygène ou NH,
A⁻ représente un anion minéral ou organique monovalent tel qu'un halogénure, un sulfate, ou un carboxylate.

6. Composition selon la revendication 5, **caractérisée en ce que** ladite silicone est de formule (XXII) ou (XXIII).

7. Composition selon les revendications 5 ou 6, **caractérisée en ce que** ladite silicone répond à la formule générale (XXIII) dans laquelle au moins l'une des conditions suivantes sont satisfaites :
- c est égal à 0;
- d désigne 0 ;
- a est égal à zéro ;
- b est égal à 1 ;
- n varie de 0 à 100 ;
- q est égal à 0 ;
- f=3;
- g = 1 ;
- R₆ et R₇ désignent le groupe méthyle ; et
- R₈ désigne un radical alkyle C₁₀-C₂₂.

8. Composition selon l'une quelconque des revendications précédentes, **caractérisée en ce que** ladite silicone est le Quaternium-80.

9. Composition selon l'une quelconque des revendications précédentes, dans laquelle la silicone à groupements ammonium quaternaire est présente en une teneur allant de 0,1 à 20 % en poids, de préférence de 0,2 à 10 % en poids, mieux de 0,3 à 5 % en poids par rapport au poids total de la composition.

10. Composition selon l'une quelconque des revendications précédentes, dans laquelle le ou les polymères non siliconés sont choisis parmi les polymères épaississants, fixants ou conditionneurs, non ioniques, anioniques, cationiques ou amphotères.

11. Composition selon la revendication précédente, dans laquelle le ou les polymères épaississants sont choisis parmi les polymères à motifs sucre associatifs ou non, les polymères anioniques acryliques ou méthacryliques associatifs ou non et les polyuréthanes associatifs ou non.

12. Composition selon la revendication 10, dans laquelle le ou les polymères conditionneurs sont choisis parmi les dérivés d'éther de cellulose quaternaires, les cyclopolymères cationiques, en particulier les homopolymères ou copolymères de chlorure de diméthyldiallylammonium, les gommes de guar modifiées cationiques, les polymères quaternaires de vinylpyrrolidone et de vinylimidazole, les homopolymères de sels de méthacrylate d'éthyltriméthylammonium et leurs mélanges.

13. Composition selon l'une quelconque des revendications précédentes, dans laquelle le ou les polymères non siliconés sont présents en une teneur allant de 0,01 à 20 % en poids, de préférence de 0,1 à 10 % en poids, mieux de 0,2 à 5 % en poids, par rapport au poids total de la composition.

14. Procédé de traitement des cheveux consistant à appliquer sur les cheveux au moins une composition capillaire selon l'une quelconque des revendications précédentes, puis éventuellement à effectuer un rinçage.

15. Procédé selon la revendication 14, comportant en outre l'étape consistant à soumettre les cheveux à un post-traitement, après traitement à l'aide d'une composition capillaire selon l'une quelconque des revendications 1 à 13, le post-traitement étant choisi parmi l'application d'un conditionneur, d'une permanente, d'un défrisant, d'un produit de coloration ou de décoloration des cheveux.

16. Procédé selon l'une quelconque des revendications 14 et 15, l'étape de traitement des cheveux étant effectuée en frottant les cheveux avec les mains nues ou en interposant au moins une surface entre les mains et les cheveux chargés de composition capillaire.

17. Kit de traitement des cheveux comportant :
- une composition capillaire comprenant au moins des particules de pierre ponce, une ou plusieurs silicones à groupements ammonium quaternaire et un ou plusieurs polymères non siliconés et
- un support comportant des instructions pour l'emploi de la composition capillaire sur les cheveux.

18. Kit selon la revendication 17, comportant en outre une composition de post-traitement des cheveux, de préférence choisie parmi un conditionneur, une permanente, un défrisant, un produit de coloration ou de décoloration des cheveux.

## Patentansprüche

1. Kapillarzusammensetzung umfassend mindestens Bimssteinteilchen, ein oder mehrere Silikone mit quaternären Ammoniumgruppen und ein oder mehreren nichtsilikonisierte Polymere.

2. Zusammensetzung nach Anspruch 1, wobei die Bimssteinteilchen einen Volumenmittel-Durchmesser von kleiner oder gleich 500 µm, vorzugsweise von 50 bis 500 µm, stärker bevorzugt von kleiner oder gleich 300 µm, beispielsweise 100 bis 300 µm, aufweisen.

3. Zusammensetzung nach einem der vorhergehenden Ansprüche, wobei die Bimssteinteilchen in einem Gehalt von 0,1 Gew.-% bis 35 Gew.-%, insbesondere von 5 Gew.-% bis 30 Gew.-%, speziell von 10 Gew.-% bis 25 Gew.-%, beispielsweise von 15 Gew.-% bis 20 Gew.-%, stärker bevorzugt von 18 Gew.-% bis 20 Gew.-%, bezogen auf das Gesamtgewicht der Zusammensetzung, vorhanden sind.

4. Zusammensetzung nach einem der vorhergehenden Ansprüche, wobei die Bimssteinteilchen eine Härte von 3 bis 10 Mohs, sogar auch größer oder gleich 4, beispielsweise größer oder gleich 5, und insbesondere von 5 bis 5,5, auf der Mohs-Skala aufweisen.

5. Zusammensetzung nach einem der vorhergehenden Ansprüche, wobei das Silikon mit quaternären Ammoniumgruppen aus den Verbindungen ausgewählt ist, die den folgenden allgemeinen Formeln entsprechen: 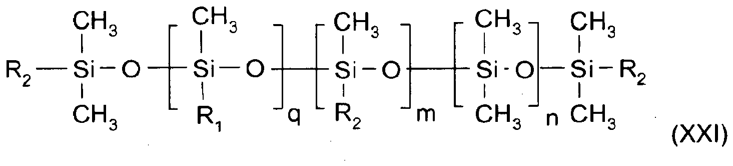 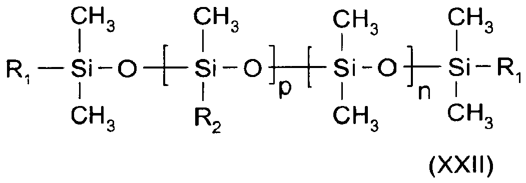 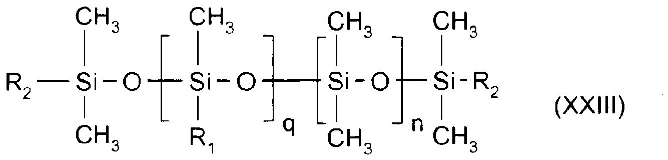 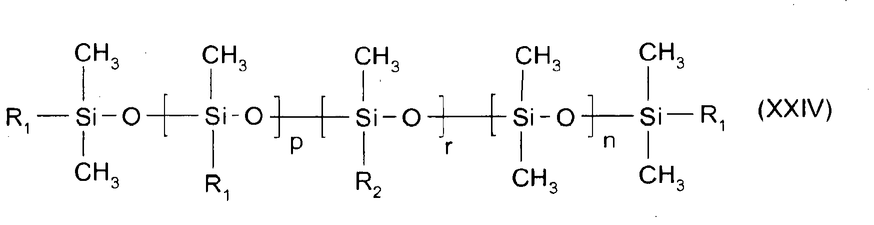 wobei:
R₁, gleich oder verschieden, eine lineare oder verzweigte C₁₋₃₀-Alkylgruppe oder Phenyl darstellt;
R₂, gleich oder verschieden, C_{c}H_{2c}-O-(C₂H₄O)ₐ-(C₃H₆O)_{b}-(PO₃H)_{d}-R₅ oder C_{c}H_{2c}-O-(C₄H₈O)ₐ-(PO₃H)_{d}-R₅ darstellt;
R₅, gleich oder verschieden, aus den Gruppen der folgenden Formel ausgewählt ist: 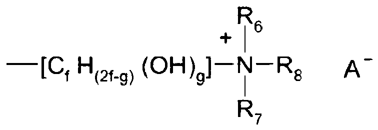
die Reste R₈ unabhängig einen C₁₋₂₂-Alkylrest oder C₂₋₂₂-Alkenylrest, linear oder verzweigt und gegebenenfalls eine oder mehrere OH-Gruppen tragend, darstellen, oder eine Gruppe CₕH₂ₕZCOR₉ darstellen; und
R₆, R₇ und R₉, gleich oder verschieden, C₁₋₂₂-Alkyl- oder C₂₋₂₂-Alkenylreste, linear oder verzweigt, gegebenenfalls eine oder mehrere OH-Gruppen tragend, darstellen, oder R₇ mit einem Teil von R₈ einen Heterocyclus (Cyclus mit mindestens einem Heteroatom, wie zum Beispiele N, O, P) bilden können, der Heterocyclus insbesondere ein Imidazolin ist,
- m von 0 bis 20 variiert;
- n von 0 bis 500 variiert;
- p von 1 bis 50 variiert;
- q von 0 bis 20 variiert;
- r von 1 bis 20 variiert;
- a von 0 bis 50 variiert;
- b von 0 bis 50 variiert
- c von 0 bis 4 variiert;
- d 0 oder 1 bezeichnet;
- f von 0 bis 4 variiert;
- g von 0 bis 2 variiert, vorzugsweise gleich 1 ist; und
- h von 1 bis 4 variiert, vorzugsweise gleich 3 ist,
- Z ein Sauerstoffatom oder NH darstellt,
- A⁻ ein einwertiges mineralisches oder organisches Anion, wie ein Halogenid, ein Sulfat oder ein Carboxylat, darstellt.

6. Zusammensetzung nach Anspruch 5, **dadurch gekennzeichnet, dass** das Silikon die Formel (XXII) oder (XXIII) aufweist.

7. Zusammensetzung nach den Ansprüchen 5 oder 6, **dadurch gekennzeichnet, dass** das Silikon der allgemeinen Formel (XXIII) gehorcht, wobei mindestens eine der folgenden Bedingungen erfüllt ist:
- c ist gleich 0,
- d bezeichnet 0;
- a ist gleich Null;
- b ist gleich 1;
- n variiert von 0 bis 100;
- q ist gleich 0;
- f = 3;
- g = 1;
- R₆ und R₇ bedeuten Methylgruppe; und
- R₈ bedeutet einen C₁₀₋₂₂-Alkylrest.

8. Zusammensetzung nach einem der vorhergehenden Ansprüche, **dadurch gekennzeichnet, dass** das Silikon Quaternium 80 ist.

9. Zusammensetzung nach einem der vorhergehenden Ansprüche, wobei das Silikon mit quaternären Ammoniumgruppen in einem Gehalt von 0,1 Gew.-% bis 20 Gew.-%, vorzugsweise von 0,2 Gew.-% bis 10 Gew.-%, stärker bevorzugt von 0,3 bis 5 Gew.-%, bezogen auf das Gesamtgewicht der Zusammensetzung vorhanden ist.

10. Zusammensetzung nach einem der vorhergehenden Ansprüche, wobei das oder die nicht-silikonisierten Polymere aus verdickenden, fixierenden oder konditionierenden, nichtionischen, anionischen, kationischen oder amphoteren Polymeren ausgewählt sind.

11. Zusammensetzung nach dem vorhergehenden Anspruch, wobei das oder die verdickenden Polymere aus assoziativen oder nicht-assoziativen Zucker-Polymeren, anionischen assoziativen oder nicht-assoziativen Acryl- oder Methacryl-Polymeren und assoziativen oder nicht-assoziativen Polyurethanen ausgewählt sind.

12. Zusammensetzung nach Anspruch 10, wobei das oder die konditionierenden Polymere aus quaternären Zelluloseetherderivaten, kationischen Cyclopolymeren, insbesondere Homo- oder Copolymeren von Dimethyldiallylammoniumchlorid, modifizierten kationischen Guargummen, quaternären Vinylpyrrolidon- und Vinylimidazol-Polymeren, Homopolymeren von Ethyltrimethylammoniummethacrylatsalzen und ihren Gemischen ausgewählt sind.

13. Zusammensetzung nach einem der vorhergehenden Ansprüche, wobei das oder die nicht-silikonisierten Polymere in einem Gehalt von 0,01 Gew.-% bis 20 Gew.-%, vorzugsweise von 0,1 Gew.-% bis 10 Gew.-%, stärker bevorzugt von 0,2 bis 5 Gew.-%, bezogen auf das Gesamtgewicht der Zusammensetzung, vorhanden sind.

14. Verfahren zum Behandeln von Haaren bestehend aus dem Aufbringen auf die Haare von mindestens einer Kapillarzusammensetzung nach einem der vorhergehenden Ansprüche, und anschließend gegebenenfalls aus dem Durchführen einer Spülung.

15. Verfahren nach Anspruch 14, umfassend weiterhin den Schritt des Unterziehens der Haare einer Nachbehandlung nach der Behandlung mit Hilfe einer Kapillarzusammensetzung nach einem der Ansprüche 1 bis 13, wobei die Nachbehandlung aus dem Aufbringen einen Konditionierungsmittel einer Dauerwelle, eines Entkrausers, eines Haar-Färbe- oder Haar-Entfärbeprodukts ausgewählt ist.

16. Verfahren nach einem der Ansprüche 14 und 15, wobei der Haarbehandlungsschritt durch Frottieren der Haare mit bloßen Händen oder durch Einlegen von mindestens einer Fläche zwischen Hände und Haare, die mit Kapillarzusammensetzung befrachtet sind, durchgeführt wird.

17. Haarbehandlungsset, umfassend:
- eine Kapillarzusammensetzung, umfassend mindestens Bimssteinteilchen, ein oder mehreren Silikone mit quaternären Ammoniumgruppen und ein oder mehreren nicht-silikonisierte Polymeren und
- einen Träger umfassend Anweisungen zum Gebrauch der Kapillarzusammensetzung auf den Haaren.

18. Set nach Anspruch 17, umfassend weiterhin eine Haar-Nachbehandlungszusammensetzung, die vorzugsweise aus einem Konditionierungsmittel, einer Dauerwelle, einem Entkrauser, einem Haar-Färbe- oder Haar-Entfärbeprodukt ausgewählt ist.

## Claims

1. A haircare composition comprising at least pumice stone particles, one or several silicones with quaternary ammonium groups and one or several non-silicone polymers.

2. The composition according to claim 1, the pumice stone particles having an average diameter by volume of less than or equal to 500 µm, preferably comprised between 50 and 500 µm, better less than or equal to 300 µm, for example comprised between 100 and 300 µm.

3. The composition according to any of the preceding claims, wherein the pumice stone particles are present in a content ranging from 0.1% to 35% by weight, notably from 5% to 30% by weight, in particular from 10% to 25% by weight, for example from 15% to 20% by weight, better from 18% to 20% by weight based on the total weight of the composition.

4. The composition according to any of the preceding claims, wherein the pumice stone particles have a hardness ranging from 3 to 10 Mohs, or even greater than or equal to 4, for example greater than or equal to 5, and in particular ranging from 5 to 5.5 on the Mohs scale.

5. The composition according to any of the preceding claims, wherein said silicone with quarternary ammonia in groups is selected from the corresponding compounds with the following general formulae: 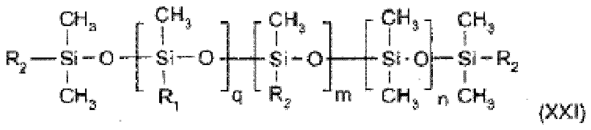 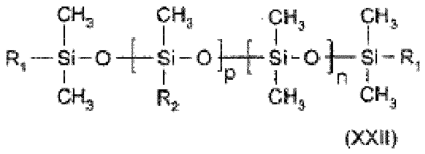 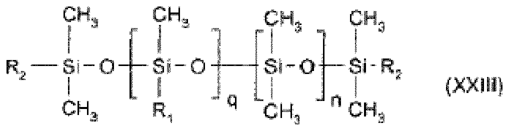 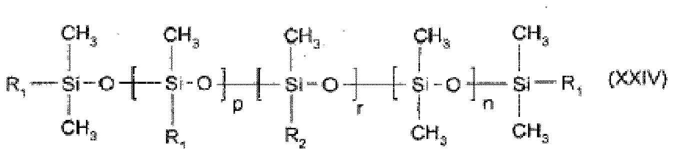 wherein:
- R₁, identical or different, represents a linear or branched C₁-C₂₀ alkyl group or phenyl group;
- R2, identical or different, represents -C_{c}H_{2c}-O-(C₂H₄O)ₐ-(C₃H₆O)_{b}-(PO₃H_{d}) -R₅ or -C_{c}H_{2c}-O-(C₄H₈O)ₐ-(PO₃H_{d}) -R₅; R₅, identical or different is selected from the groups of the following formula: 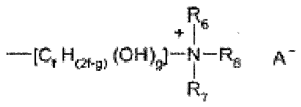
- the radicals R₈ represent independently a linear or branched C₁-C₂₂ alkyl or C₂-C₂₂ alkenyl radical, and optionally bearing one or several OH groups, or represent a group CₕH₂ₕZCOR₉; and
- R₆, R₇ and R₉, either identical or different, represent linear or branched C₁-C₂₂ alkyl or C₂-C₂₂ alkenyl radicals, optionally bearing one or several OH groups, or R₇ may form with a portion of R₈ a heterocycle (a ring with at least one heteroatom such as for example N, 0, P), the heterocycle is notably an imidazoline,
- m varies from 0 to 20;
- n varies from 0 to 500;
- p varies from 1 to 50;
- q varies from 0 to 20;
- r varies from 1 to 20;
- a varies from 0 to 50;
- b varies from 0 to 50;
- c varies from 0 to 4;
- d designates 0 or 1;
- f varies from 0 to 4;
- g varies from 0 to 2, is preferably equal to 1; and
- h varies from 1 to 4, is preferably equal to 3,
Z represents an oxygen atom or NH,
A⁻ represents a monovalent mineral or organic anion such as a halide, a sulfate or a carboxylate.

6. The composition according to claim 5, characterizing that said silicone is of formula (XXII) or (XXIII).

7. The composition according to claims 5 or 6, **characterized in that** said silicone fits the general formula (XXIII), wherein at least one of the following conditions are met:
- c is equal to 0;
- d designates 0;
- a is equal to 0;
- b is equal to 1;
- n varies from 0 to 100;
- q is equal to 0;
- f = 3;
- g = 1;
- R₆ and R₇ designate the methyl group; and
- R₈ designates a C₁₀-C₂₂ alkyl radical.

8. The composition according to any of the preceding claims, **characterized in that** said silicone is Quaternium-80.

9. The composition according to any of the preceding claims, wherein the silicone with the quaternary ammonium groups is present in a content ranging from 0.1 to 20% by weight, preferably from 0.2 to 10% by weight, better from 0.3 to 5% by weight based on total weight of the composition.

10. The composition according to any of the preceding claims, wherein the non-silicone polymer(s) is(are) selected from thickening, fixing or conditioning, non-ionic, anionic, cationic or amphoteric polymers.

11. The composition according to the preceding claim, wherein the thickening polymer(s) is(are) selected from polymers with sugar units, either associative or not, acrylic or methacrylic anionic polymers, either associative or not, and polyurethanes, either associative or not.

12. The composition according to claim 10, wherein the conditioning polymer(s) is(are) selected from quaternary cellulose ether derivatives, cationic cyclopolymers, in particular homopolymers or copolymers of dimethyldiallylammonium chloride, cationic modified guar gums, quaternary polymers of vinylpyrrolidone and vinylimidazole, homopolymers of ethyltrimethylammonium methacrylate salts and mixtures thereof.

13. The composition according to any of the preceding claims, wherein the non-silicone polymer(s) is(are) present in a content ranging from 0.01 to 20% by weight, preferably from 0.1 to 10% by weight, or better from 0.2 to 5% by weight, based on the total weight of the composition.

14. A method for treating hair consisting of applying on the hair a haircare composition according to any of the preceding claims and then optionally performing a rinse.

15. The method according to claim 14, further including the step consisting of submitting the hair to a post-treatment, after treatment with a haircare composition according to any of claims 1 to 13, the post-treatment being selected from the application of a conditioner, of a perm, of a relaxer, of a product for coloring or discoloring the hair.

16. The method according to any of claims 14 and 15, the hair treatment step being carried out by rubbing the hair with naked hands or by interposing at least one surface between the hands and the hair loaded with haircare composition.

17. A kit for treating hair including:
- a haircare composition comprising at least pumice stone particles, one or several silicones with quaternary ammonium groups and one or several non-silicone polymers and
- a support including instructions for using the haircare composition on the hair.

18. The kit according to claim 17, further including a hair post-treatment composition, preferably selected from a conditioner, a perm, a relaxer, a product for coloring or discoloring hair.
